## Europäisches Patentamt

## European Patent Office

## Office européen des brevets

(11) Veröffentlichungsnummer: **0 332 952 A2**

(12) **EUROPÄISCHE PATENTANMELDUNG**

(21) Anmeldenummer: 89103744.2

(22) Anmeldetag: 03.03.89

(51) Int. Cl.4: **A61K 45/06 , A61K 31/725 , A61K 37/02 , A61K 39/395 , A61K 45/02 , //(A61K31/725, 31:70,31:66,31:415,31:19, 31:045,31:12),(A61K45/06, 31:725),(A61K37/02,31:725), (A61K39/395,31:725), (A61K45/02,31:725)**

Patentansprüche für folgenden Vertragsstaat: ES.

(30) Priorität: 12.03.88 DE 3808353

(43) Veröffentlichungstag der Anmeldung: 20.09.89 Patentblatt 89/38

(84) Benannte Vertragsstaaten:
AT BE CH DE ES FR GB IT LI NL

(71) Anmelder: **BASF Aktiengesellschaft**
**Carl-Bosch-Strasse 38**
**D-6700 Ludwigshafen(DE)**

(72) Erfinder: **Daum, Lothar, Dr.**
**Reiherstrasse 25**
**D-6701 Otterstadt(DE)**
Erfinder: **Doerper, Thomas, Dr.**
**Luitpoldstrasse 3**
**D-6719 Bissersheim(DE)**
Erfinder: **Geiss, Karl-Heinz, Dr.**
**Eckbachring 46**
**D-6711 Heuchelheim(DE)**
Erfinder: **Herr, Dieter, Dr.**
**Hardenburgstrasse 19**
**D-6701 Altrip(DE)**
Erfinder: **Schlick, Erich, Dr.**
**9 Reiherstrasse**
**D-6701 Otterstadt(DE)**

(54) **Kombination von polysulfatierten Heparinen bei der Bekämpfung von Retrovirusinfektionen.**

(57) Kombinationen von polysulfatierten Heparinen mit Hemmstoffen der reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden zur Prophylaxe und Therapie von durch Retroviren verursachten Krankheiten.

## Kombinationen von polysulfatierten Heparinen bei der Bekämpfung von Retrovirusinfektionen

Eine Vielzahl bösartiger lymphatisch-leukämischer Neoplasien bei Mäusen, Rindern, Vögeln, Affen und Menschen werden durch Retroviren hervorgerufen. Insbesondere wird die erworbene Immunschwäche (AIDS) beim Menschen durch ein Retrovirus, das HIV (human immunodeficiency virus), verursacht.

Ansätze zur prophylaktischen und therapeutischen Chemobehandlung der genannten Virusinfektionen beim Menschen und anderen Säugern sind die Hemmung der Virusvermehrung durch Hemmstoffe der Reversen Transkriptase, durch Hemmstoffe der Bindung der Retroviren an die Wirtszeile, durch Hemmstoffe der Virusbiosynthese, durch antiviral wirksame Lymphokine sowie durch Retinoide.

In der noch nicht publizierten deutschen Patentanmeldung 37 44 119 wurde die Verwendung von polysulfatierten Heparinen zur Prophylaxe und Therapie von durch Retroviren verursachten Krankheiten beschrieben.

Gegenstand der Erfindung ist die Kombination von polysulfatierten Heparinen mit Hemmstoffen der reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden.

Weiterer Gegenstand der Erfindung ist die Verwendung von Kombinationen von polysulfatierten Heparinen mit Hemmstoffen der reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden zur Prophylaxe und Therapie von durch Retroviren verursachten Krankheiten.

Die folgenden Beispiele geben bevorzugte Bestandteile für die Kombination an.

Beispiele für Hemmstoffe der Reversen Transkriptase sind:
$3'$-Azido-$2',3'$-dideoxythymidin, $2',3'$-Dideoxycytidin, $2',3'$-Dideoxythymidin, $2',3'$-Dideoxyinosin, $2',3'$-Dideoxycytidinen, $2',3'$-Dideoxyadenosin, $3'$-Azido-$2',3'$-dideoxyadenosin, $3'$-Azido-$2',3'$-dideoxyuridin sowie Foscarnet (vgl. Trends in Pharmacological Science, Vol. 8 (1987), 339-345; J. Med. Chem. 30 (1987), 1270-1278 und dort zitierte Literatur).

Beispiele für Hemmstoffe der Bindung von Retroviren an die Wirtszellen sind Diphenylhydantoin und (4,4$'$-Dihydroxydiphenyl)-hydantoin (EP-OS 236 951), Peptid T (EP-OS 249 390) sowie monoklonale Antikörper gegen $T_4$-Rezeptoren wie z. B. OKT₄ (Nature 325 (1987) 773-778).

Beispiele der Hemmstoffe der Virusbiosynthese sind Avarol und Avaron (J. Natl. Cancer Inst. 78 (1987), 663-666).

Beispiele für antiviral wirksame Lymphokine sind Interferon $\alpha$, Interferon $\beta$, Interferon $\gamma$ (Antiviral Research 6 (1986), 1-17), Tumor Necrosis Factor $\alpha$ (TNF$\alpha$) und Lymphotoxin (TNF $\beta$) (Nature 323 (1986), 819-822).

Beispiele für Retinoide sind all-trans-Retinsäure, 13-cis-Retinsäure, Retinol, Etretinate, 4-Hydroxiphenylretinamid, Temaroten, Acitretin, all-trans-3,7,11,15-Tetramethyl-2,4,6,10,14-hexadecapentaensäure, 1,2,3,4-Tetrahydro-1,1,4,4-tetramethyl-6-(1-methyl-2-(4-hydroxy-phenyl))-ethenylnaphthalin und 1,2,3,4-Tetrahydro-1,1,4,4-tetra-methyl-6(1-methyl-2-(4-ethylsulfonylpheny l))-ethenylnaphthalin.

Ganz besonders eignen sich für Kombinationen mit polysulfatierten Heparinen $3'$-Azido-$2',3'$-dideoxythymidin, $2',3'$-Dideoxycytidin, Foscarnet, Diphenylhydantoin, (4,4$'$-Dihydroxydiphenyl)-hydantoin, OKT₄, Avarol, Avaron, Interferon-$\beta$, Tumor Necrosis Factor $\alpha$ und $\beta$, Retinol, all-trans-Retinsäure, 13-cis-Retinsäure, Aciretin und 4-Hydroxiphenylretinamid.

Polysulfatiertes Heparin ist Heparin, welches an beliebigen freien Hydroxy- und Aminogruppen zusätzlich sulfatiert ist, so daß der Schwefelgehalt bei mindestens 13 % liegt. Das erfindungsgemäß verwendete polysulfatierte Heparin besitzt vorzugsweise einen Schwefelgehalt von 13 bis 16 %. Polysulfatierte Heparine mit einem Schwefelgehalt von über 16 % sind praktisch nicht herstellbar.

Das polysulfatierte Heparin kann als solches oder in Form seiner Salze mit physiologisch verträglichen Basen verwendet werden. Als Salze sind insbesondere die Na-, Ca- und Mg-Salze zu verstehen. Auch Salze mit organischen Basen wie Diethylamin, Triethylamin oder Triethanolamin kommen in Betracht. Der Ausdruck "polysulfatiertes Heparin" - wie er hier verwendet wird - schließt die Salze mit ein.

Das Heparin, welches polysulfatiert wird, kann natürlich vorkommendes Heparin sein, welches ein Molekulargewicht im Bereich von 10 000 bis 40 000 Dalton besitzt. Durch Behandlung des natürlich vorkommenden Heparins mit Nitrit (Biochemistry 15, (1976) 3932) oder mit einer Mischung aus Schwefelsäure und Chlorsulfonsäure (FR-PS 2 538 404) erhält man Heparine mit geringeren Molekulargewichten. Polysulfatierte Heparine mit einem Molekulargewicht von unter 1000 eignen sich nur noch schlecht zur Bekämpfung von Retroviren. Am besten geeignet sind polysulfatierte Heparine mit einem Molekulargewicht von etwa 2000 bis 20 0000. Die polysulfatierten Heparine mit einem Molekulargewicht von 2000 bis 9000 haben darüber hinaus den Vorteil, daß sie oral appliziert werden können.

Die für die Kombination verwendeten polysulfatierten Heparine haben einen Schwefelgehalt von 13,0-16,0 %, bevorzugt von 13 % bis 15 % sowie ein Molgewicht von 2 000-40 000, bevorzugt von 3 000 bis 14 000.

Polysulfatierte Heparine hemmen die Reverse Transkriptase und hemmen die Replikation von Retroviren, insbesondere von HIV-I-Viren.

Durch die Kombination von polysulfatierten Heparinen mit Hemmstoffen der Reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden wird die Hemmung der Virusreplikation in überadditiver Weise verstärkt.

Diese Wirkungsverstärkung kann durch die Kombination von einem Teil (Teil = Gewichtsteil) eines polysulfatierten Heparins mit

$0,2 \cdot 10^{-3}$ bis $2 \cdot 10^{-3}$ Teilen 3'-Azido-2',3'-dideoxythymidin,

$4 \cdot 10^{-3}$ bis $4 \cdot 10^{-2}$ Teilen 2',3'-Dideoxycytidin,

1 bis 20 Teilen Diphenylhydantoin,

0,1 bis 2 Teilen Avarol,

0,1 bis 2 Teilen Avaron,

0,05 bis 5 Teilen all-trans-Retinsäure bzw. einem anderen Retinoid

0,05 bis 2 Teilen Tumor Necrosis Factor alpha

0,01 bis 1 Teilen Lymphotoxin

0,05 bis 5 Teilen Interferon-gamma

1 bis 100 Teilen $OKT_4$

erhalten werden.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,1 und 2 mg/kg Körpergewicht bei parenteraler Gabe und zwischen 1-10 mg/kg Körpergewicht bei oraler Gabe. Im Normalfall werden mit täglichen Dosen von 3 mg/kg oral und 0,5 mg/kg parenteral zufriedenstellende Ergebnisse erzielt.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gewichtsprozent.

Beispiel 1A

Herstellung von polysulfatiertem Heparin

10 g unfraktioniertes Heparin oder 10 g depolymerisiertes niedermolekulares Heparin werden in 100 ml $H_2O$ gelöst und über eine Säule mit 50 ml Kationenaustauscher (z.B. DOWEX® MSC-1) gegeben und mit $H_2O$ eluiert. Im Eluat erscheint die entsprechende freie Heparinsäure. Diese wird sodann mit ca. 30 ml Pyridin auf pH 7 neutralisiert und anschließend lyophilisiert. Die Ausbeute beträgt ca. 11 g Heparin-Pyridinium-Salz.

Die nach obiger Methode hergestellten Pyridinium-Salze diverser Heparine stellen das zur Polysulfatierung eingesetzte Ausgangsmaterial dar. Die Intensität der Sulfatierung ist hierbei abhängig von der Menge der eingesetzten Chlorsulfonsäure. Die folgende Vorschrift soll die Herstellung diverser polysulfatierter Heparine näher erläutern.

10 g Heparin-Pyridinium-Salz wurden in 50 ml Pyridin unter Einleitung von Stickstoff eingerührt und auf 50 °C erwärmt. Separat wurden 20 ml Chlorsulfonsäure (2 ml/g Heparin-Pyridinium-Salz) unter Rühren in 300 ml Pyridin eingetropft. Die Temperatur dieses Reaktionsansatzes betrug 90 °C. Nach kurzem Rühren wurde die Pyridin-Chlorsulfonsäuremischung in die vorgelegte Heparin-Suspension unter heftigem Rühren eingebracht. Danach wurden weitere 60 min bei 50 °C nachgerührt und auf Raumtemperatur abgekühlt.

Der körnige Niederschlag wurde abfiltriert, mit Methanol nachgewaschen, in 100 ml 0,5 % NaCl-Lösung aufgenommen und mit 600 ml Ethanol ausgefällt. Der erhaltene Niederschlag wurde in $H_2O$ gelöst und das Pyridin-Heparin über 80 ml Kationenaustauscher DOWEX® MSC-1 wiederum in die freie Heparinsäure überführt. Diese wurde sodann mit 10 N NaOH auf pH 7 eingestellt und mit dem 6-fachen Volumen an Ethanol ausgefällt. Das Sediment wurde in 100 ml $H_2O$ aufgenommen, filtriert und gefriergetrocknet. Die Ausbeute betrug 10,8 g, das erhaltene Produkt (A) hatte ein Molekulargewicht ($\overline{MW}$) von 14 200 Dalton und einen Schwefelgehalt von 14,8 %.

Analog wurden Produkte mit folgenden Eigenschaften hergestellt:

1B. ( $\overline{MW}$ ) = 8.200, Schwefelgehalt 14,9 % (aus Heparin und dem Molekulargewicht von 7.700 Dalton)

1C. ( $\overline{MW}$ ) = 5.600, Schwefelgehalt 14,1 % (aus Heparin und dem Molekulargewicht von 5.100 Dalton)

1D. ( $\overline{MW}$ ) = 4.300, Schwefelgehalt 13,6 % (aus Heparin und dem Molekulargewicht von 3.800 Dalton)

1E. ( $\overline{MW}$ ) = 2.800, Schwefelgehalt 14,7 % (aus Heparin und dem Molekulargewicht von 2.500 Dalton)

Beispiel 2

Auf einer Tablettenpresse werden in üblicher Weise Tabletten folgender Zusammensetzung gepreßt:
100 mg Substanz des Beispiels 1A
50 mg 3'-Azido-2',3'-didesoxythymidin
120 mg Maisstärke
13,5 mg Gelatine
45 mg Milchzucker
2,25 mg Aerosil®
(chemisch reine Kieselsäure in submikroskopisch feiner Verteilung)
6,75 mg Kartoffelstärke (als 6 %iger Kleister)

Beispiel 3

Herstellung von Dragees

In üblicher Weise werden Dragees folgender Zusammensetzung hergestellt:
150 mg Substanz des Beispiels 1D
50 mg Avarol
60 mg Kernmasse
60 mg Verzuckerungsmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 4

Herstellung einer Lösung

7 g Substanz des Beispiels 1B und 3 g TNFα werden in 5000 ml Wasser unter Zusatz von NaCl gelöst und mit 0,1 N NaOH auf pH 6,0 eingestellt, so daß eine blutisotonische Lösung entsteht die steril filtriert wird. Jeweils 5 ml dieser Lösung werden in Ampullen gefüllt.

**Ansprüche**

1. Kombination von polysulfatierten Heparinen mit Hemmstoffen der reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden.

2. Verwendung von Kombinationen von polysulfatierten Heparinen mit Hemmstoffen der reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden zur Prophylaxe und Therapie von durch Retroviren verursachten Krankheiten.

Patentanspruch für folgenden Vertragsstaat : ES

Verfahren zur Herstellung eines Arzneimittels gegen Retroviren, dadurch gekennzeichnet, daß man polysulfatierte Heparine mit Hemmstoffen der reversen Transkriptase, und/oder mit Hemmstoffen der Bindung von Retroviren an die Wirtszelle, und/oder Hemmstoffen der Virusbiosynthese, und/oder mit antiviral wirkenden Lymphokinen, und/oder mit Retinoiden mischt und anschließend zu einer Applikationsform verarbeitet.